# NEW EUROPEAN PATENT SPECIFICATION

(11) **EP 2 675 838 B2**
(45) Date of publication and mention of the opposition decision: **15.05.2024**
(45) Mention of the grant of the patent: 02.12.2020
(21) Application number: 12705393.2
(22) Date of filing: 14.02.2012
(51) Int. Cl.: C08K 3/22, C08K 3/24, C08J 9/00, C08L 71/12, A61L 27/46, C08J 9/26, C08J 3/20, C08K 3/32

(54) **COMPONENTS INCORPORATING BIOACTIVE MATERIAL**
KOMPONENTEN MIT BIOAKTIVEM MATERIAL
COMPOSANTS COMPRENANT UN MATÉRIAU BIOACTIF

(30) Priority: 14.02.2011 GB 201102561
(43) Date of publication of application: 25.12.2013
(73) Proprietor: Invibio Limited, Lancashire FY5 4QD (GB)
(72) Inventor: JARMAN-SMITH, Marcus, Thornton Cleveleys Lancashire FY4 5QD (GB); BRADY, Mark, Thornton Cleveleys Lancashire FY5 4QD (GB); DEVINE, John, Thornton Cleveleys Lancashire FY5 4QD (GB); CARTWRIGHT, Keith, Thornton Cleveleys Lancashire FY5 4QD (GB)
(74) Representative: Appleyard Lees IP LLP
(86) International application number: PCT/GB2012/050331
(87) International publication number: WO 2012/110803

(56) References cited:
- FR-A1- 2 722 694
- GB-A- 2 469 991
- JP-A- 2010 035 827
- US-A- 5 092 890
- US-A- 5 872 159
- US-A1- 2002 115 742
- US-A1- 2008 206 297
- US-B2- 7 230 039
- WANG L ET AL: "Mechanical properties and microstructure of polyetheretherketone-hydr oxyapatite nanocomposite materials", MATERIALS LETTERS, vol. 64, no. 20, 31 October 2010 (2010-10-31), pages 2201-2204, XP027229484, NORTH HOLLAND PUBLISHING COMPANY. AMSTERDAM, NL ISSN: 0167-577X [retrieved on 2010-07-01]
- WONG K L ET AL: "Mechanical properties and in vitro response of strontium-containing hydroxyapatite/polyetheretherketone composites", BIOMATERIALS, vol. 30, no. 23-24, 1 August 2009 (2009-08-01), pages 3810-3817, XP026149146, ELSEVIER SCIENCE PUBLISHERS BV., BARKING, GB ISSN: 0142-9612, DOI: 10.1016/J.BIOMATERIALS.2009.04.016 [retrieved on 2009-05-07]
- ABU BAKAR M S ET AL: "Tensile properties, tension-tension fatigue and biological response of polyetheretherketone-hydroxyapatite composites for load-bearing orthopedic implants", BIOMATERIALS, vol. 24, no. 13, 1 June 2003 (2003-06-01), pages 2245-2250, XP004420014, ELSEVIER SCIENCE PUBLISHERS BV., BARKING, GB ISSN: 0142-9612, DOI: 10.1016/S0142-9612(03)00028-0
- TANG S M ET AL: "Tension-tension fatigue behavior of hydroxyapatite reinforced polyetheretherketone composites", INTERNATIONAL JOURNAL OF FATIGUE, vol. 26, no. 1, 1 January 2004 (2004-01-01), pages 49-57, XP009157711, BUTTERWORTH SCIENTIFIC LTD, GUILDFORD, GB ISSN: 0142-1123
- DATABASE WPI Week 201014 Thomson Scientific, London, GB; AN 2010-B82423 XP002672144, -& JP 2010 035827 A (NGK SPARK PLUG CO LTD) 18 February 2010 (2010-02-18)
- CONVERSE G L ET AL: "Tensile properties of hydroxyapatite whisker reinforced polyetheretherketone", MATERIALS RESEARCH SOCIETY SYMPOSIUM PROCEEDINGS, vol. 898, 1 January 2005 (2005-01-01), page 6PP, XP009157765, MATERIALS RESEARCH SOCIETY, USA ISSN: 0272-9172
- BOHNER M ET AL: "Can bioactivity be tested in vitro with SBF solution?", BIOMATERIALS, vol. 30, no. 12, 1 April 2009 (2009-04-01) , pages 2175-2179, XP025990542, ELSEVIER SCIENCE PUBLISHERS BV., BARKING, GB ISSN: 0142-9612, DOI: 10.1016/J.BIOMATERIALS.2009.01.008 [retrieved on 2009-01-26]
- DATABASE WPI Week 200765 Thomson Scientific, London, GB; AN 2007-691349 XP002672145, & CN 1 943 800 A (UNIV QINGHUA) 11 April 2007 (2007-04-11)
- Biomaterials 28 (2007) 4845^4869, 2007
- Abstract "Mechanical Properties and Bioactivity of PEEKOPTIMA ®/Hydroxyapatite Compounds" - 2011
- Brady M, Et Al: "Mechanical Properties and Bioactivity of PEEK-OPTIMA Hydroxyapatite Compounds abstract #761", 35th Annual Meeting of the Society for Biomaterials, 16 April 2011 (2011-04-16), - 16 April 2011 (2011-04-16), pages 1-1,
- Abu Bakar, M.S. ; Cheang, P. ; Khor, K.A.: "Mechanical properties of injection molded hydroxyapatite-polyetheretherketone biocomposites", Composites Science and Technology, ELSEVIER, AMSTERDAM, NL, vol. 63, no. 3-4, 1 February 2003 (2003-02-01), pages 421-425, AMSTERDAM, NL ISSN: 0266-3538
- REVIEW OF POTENTIAL PROCESSING TECHNIQUES FOR THEENCAPSULATION OF WASTES IN THERMOPLASTIC POLYMERS1995
- Medical Plastics 2003, vol 17, C 18 1-C 18.14
- Polymer 2007, 48.598-615
- Mech Time -Depend Mater (2014) 18 663-684
- Journal of Materials Processing Technology 89-90 (1999) 462-466

## Description

This invention relates to components and particularly, although not exclusively, relates to bioactive components for use as medical implants or parts thereof and to methods of manufacturing components.

PEEK (polyetheretherketone) is widely used as a medical implant material due to its biocompatibility, advantageous mechanical properties and high chemical resistance. Attempts have been made to compound PEEK with bioactive materials such as HA (hydroxyapatite) to further improve bone fixation.

M.S. Abu Bakar et al. Composites Science and Technolgy 63 (2003) 421-425 discloses compounding of HA and PEEK composites in a mixer as a batch and these were then granulated and dried before being injection moulded. However, this reports weak interaction at the HA-PEEK interface. There are numerous other proposals for incorporating PEEK and HA to produce bioactive materials. However, known compounds have poor physical properties, in particular tensile properties, and/or the bioactivity is low.

With known proposals for producing HA and PEEK composite components compromise is observed with respect to the mechanical properties and potential for scalable production.

It is an object of embodiments of the present invention to address problems associated with bioactive components and/or the manufacture of such components.

According to a first aspect of the present invention there is provided a method according to claim 1.

According to a further aspect, the invention provides pellets according to claim 14.

Suitably, the bioactive material (II) comprises a material selected from the group consisting of apatites and calcium phosphates.

Suitably, the bioactive material consists of an apatite and/or calcium phosphate. Suitably, the bioactive material consists of an apatite. Suitably, the bioactive material consists of hydroxyapatite (HA).

Suitably, as used herein the term "bioactive component" refers to a component incorporating a "bioactive material" such that the component has one or more bioactive characteristics associated with the bioactive material and wherein said bioactive material is suitably as defined herein.

Suitably, the bioactive material is a material which elicits a specific biological response at the interface of the material, which results in a formation of a bond between tissue and said bioactive material.

Suitably, the component comprises a bioactive component for clinical use. Suitably, the component comprises an implant adapted for bioactive fixation. Suitably, as used herein the term "bioactive fixation" refers to interfacial bonding of an implant to tissue by means of formation of a biologically active hydroxyapatite layer on the implant surface.

Suitably, the component is a bioactive component which is adapted such that bone-like apatite may form on its surface when implanted in a living body.

Suitably, the component is adapted to bond to hard tissue. The component may be adapted to bond to hard and/or soft tissue. The component may be adapted to bond only to hard tissue.

Suitably, the component is a component which, when placed in a simulated body fluid (SBF) test for bioactivity, passes said test with the formation of new apatite (CaP) at the ratio close to the theoretical value for hydroxyapatite, which is 1.67. For example with the formation of new apatite (CaP) at the ratio of 1.66. Suitably, said SBF test is performed according to the method described in. Bohner and Lemaitre (Bohner M, Lemaitre J. / Biomaterials 30 (2009)2175-2179).

Suitably, the SBF is a fluid with ion concentrations similar to human blood plasma and which can precipitate hydroxyapatite at the physiological temperature (37°C). Suitably, the bioactive component is a component on which bone-like hydroxyapatite will form after it is immersed in such an SBF fluid.

Suitably, the SBF test is performed using SBF-JL2 as prepared and described in Bohner and Lemaitre (Bohner M, Lemaitre J. / Biomaterials 30 (2009) 2175-2179) and the SBF-JL2 is thus produced using a dual-solution preparation (Sol. A and Sol.B) having the following composition for 2L of final fluid:

| Starting Materials | | | Weights of starting materials [g/L] | |
|---|---|---|---|---|
| Formula | MW [g/mol] | Purity [-] | Sol. A | Sol. B |
| NaCl | 58.44 | 99.5% | 6.129 | 6.129 |
| NaHCO₃ | 84.01 | 99.5% | 5.890 | |
| Na₂HPO₄.2H₂O | 177.99 | 99.0% | 0.498 | |
| CaCl₂ | 110.99 | 95.0% | | 0.540 |
| | | | Volume of HCl solution (mL/L) | |
| HCl 1.00 M | Aq. Sol. | [mL/L] | 0.934 | 0.934 |

The component may be such that bioactivity may be confirmed by the presence of increased bone in contact when in vivo and assessed using histology.

Suitably, the polymeric material consists of polyetheretherketone (PEEK).

Suitably, the method comprises using a twin screw extruder to mix a polymeric material (I) with a bioactive material (II) and melt the polymeric material (I).

Suitably, step (a) comprises producing discrete units of composite material, for example pellets. Suitably, step (a) comprises producing pellets by pelletizing the output from the extruder.

Suitably, step (a) comprises forming pellets having a length of 10mm or less. Suitably, step (a) comprises forming pellets having a length of 5mm or less, for example of 3.5mm or less, for example around 3.0mm. Suitably, the method comprises producing pellets having a diameter of 3.0mm or less, for example 2.5mm or less, for example around 2.0mm. Suitably, step (a) comprises forming pellets having a length of at least 0.1mm. Suitably, step (a) comprises forming pellets having a length of 0.4mm or more. Suitably, the method comprises producing pellets having a diameter of at least 0.1mm, for example at least 0.4mm.

Step (a) may alternatively comprise producing laces, for example laces having a length of 1m or more by cutting lengths from the output of the extruder.

Suitably, the method comprises producing pellets of composite material in step (a) and making a part by moulding from the pellets in step (b).

Suitably, step (b) comprises injection moulding. Suitably, step (b) comprises injection moulding from pellets produced in step (a).

Suitably, the method comprises pelletizing the output from the extruder in step (a) and subsequently melting the pellets so formed to produce a component by injection moulding in step (b). Step (b) may thus be performed immediately after step (a) or it may be performed at a later time, for example hours, days or even weeks later. Step (b) may be performed at the same location as step (a) or pellets may be transported and step (b) performed at a distinct location.

Surprisingly, it has been found that having a pellet forming stage prior to moulding may provide good mixing and good bonding between bioactive material and polymeric material as well as allowing retention of good mechanical properties whilst also allowing use of industrial scale injection moulding equipment to produce a component which is bioactive.

Surprisingly, it has been found that using a twin screw extruder to mix PEEK and HA and pelletizing the output of the extruder to form pellets which are then used to injection mould a bioactive component may result in a bioactive component which advantageously has tensile properties which are relatively close to those of PEEK whilst also having surprisingly high bioactivity. The bioactivity of components incorporating low levels of HA is particularly unexpected and, without wishing to be bound by theory, is believed to be due to the manufacturing process making significant quantities of HA available at the surface of the bioactive component.

Although twin screw extrusion is a known technique for compounding a filler with a polymeric material it has unexpectedly been found by the present inventors that using such a screw extrusion process may result in better dispersion of bioactive material such as HA through a polymeric material such as PEEK than other methods of blending. Also unexpectedly this manufacture method results in high availability of HA at the surface of the moulded bioactive component. This may allow lower levels of HA to be used so allowing other physical properties of the component to be less compromised as the polymeric material may be maintained at a high level without significantly affecting bioactivity of the component. In addition it has been found that bonding between the bioactive material such as HA and the matrix of polymeric material such as PEEK may be better than achieved in the prior art.

Suitably, the component comprises a component for medical use. Suitably, the component comprises an implant.

Suitably, the method comprises supplying an extruder with polymeric material (I) and bioactive material (II) such that they are substantially homogenously distributed in the output of the extruder.

Suitably, the method comprises producing a component in which the polymeric material (I) and bioactive material (II) are substantially homogenously distributed. Suitably, the method comprises producing a component having bioactive material (II) located at the surfaces of said component.

Suitably, the method comprises supplying an extruder with polymeric material (I) and bioactive material (II) in such ratios that the component is imparted with bioactive properties by the bioactive material whilst retaining desirable physical characteristics of the polymeric material.

Suitably, the method comprises producing a component comprising a polymeric material-bioactive material composite having tensile strength and/or flexural strength which are at least 80% of the respective strength of the polymeric material. Suitably, the method comprises producing a component comprising a polymeric material-bioactive material composite having a tensile strength which is at least 85% of the respective strength of the polymeric material. Suitably, the method comprises producing a component comprising a polymeric material-bioactive material composite having a flexural strength which is at least 90% of the respective strength of the polymeric material.

Suitably, the tensile strength is measured according to the method of ISO 527 (specimen type 1b) tested at 23°C at a rate of 50mm/minute).

Suitably, the flexural strength is measured according to the method of ISO 178 (80mm x 10mm x 4mm specimen, tested in three-point-bend at 23°C at a rate of 2mm/minute).

Suitably, the method comprises producing a component comprising a polymeric material-bioactive material having an impact strength of at least 5KJ m⁻², for example at least 6KJ m⁻². Suitably, the method comprises producing a bioactive component comprising a polymeric material-bioactive material having an impact strength of no more than 10KJ m⁻²

Suitably, the impact strength is measured according to the method of ISO 180.

Suitably, the method comprises producing a component comprising a polymeric material-bioactive material composite having a flexural strength of at least 150MPa, for example at least 155MPa.

Suitably, the method comprises producing a component comprising a polymeric material-bioactive material composite having a flexural modulus of 6GPa or less, for example 5GPa or less.

Suitably, the flexural modulus is measured according to the method of ISO 178 (80mm x 10mm x 4mm specimen, tested in three-point-bend at 23°C at a rate of 2mm/minute).

The method comprises producing a component comprising a polymeric material-bioactive material composite having a tensile strength of at least 85MPa.

Suitably, the method comprises producing a component comprising a polymeric material-bioactive material composite having a strain at break of at least 3%, suitably at least 4%, for example at least 8%.

Suitably, the strain at break is measured according to the method of ISO 180.

Suitably, the method comprises producing a bioactive component comprising a polymeric material-bioactive material composite having the ability to bond with tissue, suitably with bone.

Suitably, the method comprises producing a bioactive component comprising a polymeric material-bioactive material which, when placed in a simulated body fluid (SBF) test for bioactivity, passes said test with the formation of new apatite (CaP) at the ratio close to the theoretical value for hydroxyapatite, which is 1.67. For example with the formation of new apatite (CaP) at the ratio of 1.66. Suitably, said SBF test is performed according to the method described for SBF-JL2 in Bohner and Lemaitre, 2009 (Bohner M, Lemaitre J. / Biomaterials 30 (2009) 2175-2179.

Suitably, the method comprises producing a bioactive component comprising a polymeric material-bioactive material composite which is such that greater apatite formation at the ratio close to the theoretical value for hydroxyapatite, which is 1.67, occurs on the surface thereof than is the case for the polymeric material (I) alone when exposed to the same conditions in which apatite formation can occur.

Suitably, said apatite formation may be determined by one or a combination of the following: thin-film X-ray diffraction (TF-XRD), scanning electron microscopy (SEM), X-ray photoelectron spectroscopy (XPS), fourier transform infrared (FTIR) spectroscopy.

Suitably, the method comprises producing a bioactive component comprising a polymeric material-bioactive material composite which is such that when immersed in SBF for 1 day on a rotating platform at 37°C with 5% CO₂ and 100% humidity greater apatite formation at the Ca/P ratio close to the theoretical value for hydroxyapatite, which is 1.67, occurs on the surface thereof than is the case for the polymeric material (I) alone.

Suitably, the method comprises producing a bioactive component comprising a polymeric material-bioactive material composite which is such that when immersed in SBF for 3 days on a rotating platform at 37°C with 5% CO₂ and 100% humidity greater apatite formation at the ratio close to the theoretical value for hydroxyapatite, which is 1.67, occurs on the surface thereof than is the case for the polymeric material (I) alone.

Suitably, the method comprises producing a bioactive component comprising a polymeric material-bioactive material composite which is such that when immersed in SBF for 7 days on a rotating platform at 37°C with 5% CO₂ and 100% humidity greater apatite formation at the ratio close to the theoretical value for hydroxyapatite, which is 1.67, occurs on the surface thereof than is the case for the polymeric material (I) alone.

Suitably, the component comprises the polymeric material (I) and bioactive material (II) in an amount of at least 90% by weight of the component, for example at least 95% by weight of the component. The component may comprise the polymeric material (I) and bioactive material (II) in an amount of at least 99% by weight of the component. The component may consists of polymeric material (I) and bioactive material (II). The component may consist of PEEK and HA.

Suitably, the component comprises the bioactive material (II) in an amount of no more than 60% by weight of the component. Suitably, the component comprises the bioactive material (II) in an amount of 50% by weight or less. Suitably, the component comprises the bioactive material (II) in an amount of 45% by weight or less for example in an amount of: 40%; 35%; 30%; 25%; 20%; 15% or 10% or less.

Suitably, the component comprises the bioactive material (II) in an amount of at least 5% by weight of the component. Suitably, the component comprises the bioactive material (II) in an amount of 10% by weight or more. Suitably, the component comprises the bioactive material (II) in an amount of 15% by weight or more for example in an amount of: 20%; 25%; 30%; 35%; 40%; 45%; or 50% or more.

Suitably, the component comprises the bioactive material (II) in an amount of between 10% and 30% by weight of the component, for example between 15% and 25% by weight of the component.

Suitably, the component comprises the polymeric material (I) in an amount of no more than 95% by weight of the component. Suitably, the component comprises polymeric material (I) in an amount of 90% by weight or less. Suitably, the component comprises the polymeric material (I) in an amount of 85% by weight or less for example in an amount of: 80%; 75%; 70%; 65%; 60%; 55% or 50% or less.

Suitably, the component comprises the polymeric material (I) in an amount of at least 40% by weight of the component. Suitably, the component comprises the polymeric material (I) in an amount of 50% by weight or more. Suitably, the component comprises the polymeric material (I) in an amount of 55% by weight or more for example in an amount of: 60%; 65%; 70%; 75%; 80%; 85%; or 90% or more.

Suitably, the component comprises the polymeric material (I) in an amount of between 70% and 90% by weight of the component, for example between 75% and 85% by weight of the component.

Suitably, the component comprises polymeric material (I) in an amount of between 70% and 90% by weight of the component and bioactive material (II) in an amount of between 10% and 30% by weight of the component. The component may comprise polymeric material (I) in an amount of between 70% and 90% by weight of the component and bioactive material (II) may make up the balance of the component.

Suitably, the component comprises polymeric material (I) in an amount of between 75% and 85% by weight of the component and bioactive material (II) in an amount of between 15% and 25% by weight of the component. The component may comprise polymeric material (I) in an amount of between 75% and 85%% by weight of the component, for example 80%, and bioactive material (II) may make up the balance of the component.

Suitably, the component comprises PEEK in an amount of between 75% and 85% by weight of the component and HA in an amount of between 15% and 25% by weight of the component. The component may comprise PEEK in an amount of between 75% and 85% by weight of the component, for example 80%, and HA may make up the balance of the component. Surprisingly it has been found that a component having a selected composition around the value of 20% by weight HA and 80% by weight PEEK may have a particularly desirable balance between bioactivity and physical properties such as tensile strength.

The component may comprise bioactive material that has been modified or doped with one or more additional chemical elements. The component may comprise bioactive material that has been modified or doped with one or more metals. The component may for example comprise HA that has been modified or doped. The HA may for example be modified or doped with one or more metals. The HA may for example be modified or doped with boron, magnesium, silicate or silver.

The component may comprise one or more of silicate (SiO₄²⁻), Borate (BO₃³⁻) and Strontium (Sr²⁺).

The component may comprise a bioactive material doped with one or more of silicate (SiO₄²⁻), Borate (BO₃³⁻) and Strontium (Sr²⁺). Suitably, the total content of silicate (SiO₄²⁻), Borate (BO₃³⁻ ) and Strontium (Sr²⁺) within the bioactive material will not exceed 10% by molarity as a cumulative value. The method may comprise doping the bioactive material with one or more of silicate (SiO₄²⁻), Borate (BO₃³⁻) and Strontium (Sr²⁺).

The component may comprise a bioactive material comprising a calcium phosphate, for example HA, in which a proportion of the calcium phosphate is substituted with one or more of silicate (SiO₄²⁻), Borate (BO₃³⁻) and Strontium (Sr²⁺). Suitably, the total substitution of calcium phosphate by silicate (SiO₄²⁻), Borate (BO₃³⁻) and Strontium (Sr²⁺) will not exceed 10% by molarity as a cumulative value. Suitably, the total substitution of calcium phosphate will not exceed 10% by molarity as a cumulative value.

The component may comprise one or more of Silicon(Si), Fluorine (F), Sulphur (S), Boron (B), Strontium (Sr), Magnesium (Mg), Silver (Ag), Barium (Ba), Zinc (Zn), Sodium (Na), Potassium (K), Aluminium (Al), Titanium (Ti)and Copper (Cu).

The method may comprise introducing single or multiple elements into a bioactive material, suitably into a calcium phosphate lattice. The method may for example comprise introducing one or more of Silicon(Si), Fluorine (F), Sulphur (S), Boron (B), Strontium (Sr), Magnesium (Mg), Silver (Ag), Barium (Ba), Zinc (Zn), Sodium (Na), Potassium (K), Aluminium (Al), Titanium (Ti) and Copper (Cu).

The component may comprise a bioactive material comprising one or more of Silicon(Si), Fluorine (F), Sulphur (S), Boron (B), Strontium (Sr), Magnesium (Mg), Silver (Ag), Barium (Ba), Zinc (Zn), Sodium (Na), Potassium (K), Aluminium (Al), Titanium (Ti) and Copper (Cu).

The component may comprise a bioactive material comprising a calcium phosphate lattice, for example a HA lattice in which single or multiple elements are introduced. For example the bioactive material may comprise a calcium phosphate lattice into which one or more of Silicon(Si), Fluorine (F), Sulphur (S), Boron (B), Strontium (Sr), Magnesium (Mg), Silver (Ag), Barium (Ba), Zinc (Zn), Sodium (Na), Potassium (K), Aluminium (Al), Titanium (Ti) and Copper (Cu) are introduced.

The bioactive material may comprise HA that has been modified or doped such that it is more potent than HA alone. This may allow lower concentrations of bioactive material to be used in the component.

The component may comprise a porous material. The component may for example comprises a material which is rendered porous using salt leaching or laser sintering. The method may comprise a step of forming pores in the component, for example by a porogen leaching or laser sintering process, for example by a salt leaching process.

The component may comprise a shaped object, for example a medical implant. The component may comprise a film, filament or textile.

The component may comprise a matrix in which there is substantially homogenous distribution of bioactive material, for example substantially homogenous distribution of HA or doped/modified HA. The component may comprise a matrix in which there is homogenous distribution of bioactive material, for example homogenous distribution of HA or doped/modified HA.

The component may comprise an adjunct that may help mixing and/or bonding. The adjunct may for example comprise one or more sizing agents. The method may comprise incorporating one or more adjuncts, for example one or more sizing agents. Surprisingly it has been found that the incorporation of an adjunct may provide even distribution, better interface of bioactive material and polymeric material, effective bioactivity with low concentrations of bioactive material and retention of mechanical properties of the polymeric material.

The component may have a change in the surface energy and/or hydrophilicity of PEEK when compared to PEEK alone. Such a change in surface energy and/or hydrophilicity may be beneficial, for example for protein attachment.

Disclosed is a method of producing a bioactive component incorporating a bioactive material wherein the method comprises:
(a) producing pellets by using a twin screw extruder to mix a polymeric material (I) with a bioactive material (II) and melt the polymeric material (I) and pelletizing the output of the extruder; and
(b) making a component by moulding from the pellets; and
wherein the bioactive material (II) comprises a phosphate and the polymeric material (I) is of a type which includes:
(i) phenyl moieties;
(ii) ketone moieties; and
(iii) ether moieties.

Suitably, there is provided a method of producing a component incorporating HA wherein the method comprises:
(a) producing pellets by using a twin screw extruder to mix PEEK with HA and melt the PEEK and pelletizing the output of the extruder; and
(b) making a component by moulding from the pellets.

Suitably, there is provided a method of producing a bioactive component incorporating HA wherein the method comprises:
(a) producing pellets by using a twin screw extruder to mix PEEK with HA and melt the PEEK and pelletizing the output of the extruder; and
(b) making a component by moulding from the pellets.

Suitably, the method comprises drying the polymeric material to remove water there from prior to introducing it to the extruder.

Suitably, the method comprises introducing the polymeric material to the extruder in a solid state. Suitably, the method comprises introducing polymeric material to the extruder in the form of powder, granules, pellets or whiskers.

The method may comprise feeding polymeric material to the extruder via a hopper. The polymeric material my be warmed in the hopper and melted in the extruder.

Suitably, the method comprises introducing the bioactive material to the extruder in a solid state. Suitably, the method comprises introducing polymeric material to the extruder in the form of powder, granules, pellets or whiskers.

Suitably the method comprises introducing the bioactive material to the extruder at a point downstream of the point at which the polymeric material is introduced to the extruder.

Suitably, the method comprises introducing the bioactive material to the extruder at a point at which the polymeric material is at least partially molten. Suitably, the method comprises introducing the bioactive material to the extruder at a point at which the polymeric material is fully molten.

Suitably, the extruder comprises twin screws. Suitably, the extruder comprises screws fabricated from stainless steel. Suitably, the extruder comprises screws having a normal screw profile. Suitably, the extruder does not use continuous compression type screws. Suitably, the screws have a minimum L/D ratio of 45:1

Suitably, the extruder is a twin screw extruder having screws of between 20mm and 50mm in diameter, for example between 30mm and 40mm in diameter. Suitably, the extruder has screws of between 0.5m and 1.5m in length for example between 0.8m and 1.2m in length, for example around 1m. Suitably, the extruder has counter rotating screws. Suitably, the extruder has intermeshing screws.

Suitably, at the extrusion end the extruder has a twin hole die. Suitably, at the extrusion end the extruder has a 4mm orifice. Suitably, at the extrusion end the extruder has a pelletizer.

Suitably, the method uses a main screw rotation speed of between 150 and 250rpm.

Suitably, the size and output of the extruder are matched to obtain short residence time, for example from 3 to 12 minutes. The extruder may for example have a residence time of between 5 and 10 minutes.

Suitably, the mixture within the extruder is heated up to between 360°C and 400°C. Suitably, the extruder is heated to 400°C. Suitably, the extruder is heated using cylinder heaters.

Suitably, the polymeric material is heated as it passed through the extruder such that it is melted within the extruder and thus has a higher temperature towards the output end than towards the input end of the extruder.

Suitably, the method comprises extruding a composite of polymeric material and bioactive material, forming pellets and then injection moulding a component using said pellets.

Suitably, the method comprises extruding a composite of polymeric material and bioactive material, forming pellets and then melting those pellets and injection moulding a component using the melt.

Suitably, the method comprises injection moulding using an injection moulding machine having a heated barrel containing a screw.

Suitably, the method comprises forming pellets and heating those pellets in an injection moulding machine to form a melt which is injected into a mould. Suitably, the injection moulding machine comprises a heated barrel in which the polymeric material is melted. Suitably, the injection moulding machine comprises a screw for conveying material through the barrel to a mould tool. Suitably, said screw mixes material as it passes through the barrel. Suitably, the barrel is heated to temperatures of between 350°C and 400°C, for example between 360°C and 375°C. Suitably, the moulding tool is heated to a temperature of between 180°C and 240°C, for example between 180°C and 220°C.

Suitably, the method comprises mixing molten polymeric material with bioactive material.

Suitably, step (a) comprises heating the mixture to at least 350°C, for example at least 360°C in the extruder. Suitably, step (a) comprises heating the mixture to between 360°C and 400°C in the extruder.

Suitably, step (a) comprises heating the mixture in the extruder until the polymeric material is molten. Suitably, step (a) comprises heating the mixture in the extruder until the polymeric material is flowable.

Suitably, step (a) comprises heating the mixture in the extruder until the polymeric material is molten and then adding the bioactive material to the extruder such that the bioactive material is mixed with the molten polymeric material by the extruder. Suitably, step (a) comprises heating the mixture in the extruder until the polymeric material is flowable and then adding the bioactive material to the extruder such that the bioactive material is mixed with the flowable polymeric material by the extruder.

Suitably, step (a) comprises holding the mixture at a temperature above the melting temperature of the polymeric material for a sufficiently long period of time to permit full melting of the polymeric material and merging together of adjacent polymeric material before the composite material exits the extruder.

Suitably, step (b) comprises heating pellets of composite material until the polymeric material is molten. Suitably, step (b) comprises heating the pellets until the polymeric material is flowable.

Suitably, step (b) comprises holding the composite material at a temperature above the melting temperature of the polymeric material for a sufficiently long period of time to permit full melting of the polymeric material and merging together of adjacent polymeric material before the composite material is injected into the mould.

The method may comprise a method of making a near net shape for the bioactive component in a mould which can then be machined and/or finished to produce an end shape of the bioactive component. Suitably, the method comprises making an end shape of the component in a mould such that no machining and/or finishing is required.

Suitably, the polymeric material is in the form of powder or granules. Suitably, the bioactive material is in the form of powder or whiskers.

The method may comprise blending the polymeric material and/or bioactive material with fillers to influence properties. For example the bioactive material, suitably HA, could be doped with antimicrobial compounds (for example silver, gold and or copper) and/or it could be combined with additional bone enhancing materials (for example Boron, silica and/or Magnesium).

The method preferably comprises selecting first particles which comprise said polymeric material and selecting second particles which comprise said bioactive material. Suitably, the method comprises combining said first and second particles. Suitably, the method comprises using a screw extruder/compounder to mix said first and second particles.

Said first particles may include particles having a volume in the range 0.001 to 3mm³, preferably in the range 0.01 to 2.5mm³, more preferably in the range 0.05 to 1.0 mm³, especially 0.1 to 0.5mm³. Substantially all of said first particles may have a volume as aforesaid.

The average volume of said first particles (total volume of first particles divided by the total number of said first particles) may be at least 0.001mm³, preferably at least 0.01mm³, more preferably at least 0.1mm³. The average volume (as described) may be less than 1mm³.

Said first particles may include particles having a maximum dimension in one direction of at least 0.1mm, preferably at least 0.2mm, more preferably at least 0.3mm. The maximum dimension may be less than 2mm, preferably less than 1mm, more preferably less than 0.8mm. Suitably, substantially all particles in the mix have maximum dimensions as aforesaid.

Said second particles may include particles having a volume in the range 0.001 to 3mm³, preferably in the range 0.01 to 2.5mm³, more preferably in the range 0.05 to 1.0 mm³, especially 0.1 to 0.5mm³. Substantially all of said second particles may have a volume as aforesaid.

The average volume of said second particles (total volume of second particles divided by the total number of said second particles) may be at least 0.001mm³, preferably at least 0.01 mm³, more preferably at least 0.1mm³. The average volume (as described) may be less than 1mm³.

Said second particles may include particles having a maximum dimension in one direction of at least 0.1mm, preferably at least 0.2mm, more preferably at least 0.3mm. The maximum dimension may be less than 2mm, preferably less than 1mm, more preferably less than 0.8mm. Suitably, substantially all particles in the mix have maximum dimensions as aforesaid. The second particles may have a mean particle size of 10µm or less for example of 5µm.

The average of the maximum dimensions (sum of maximum dimensions of all particles divided by the total number of said particles) may be at least 0.1mm, preferably at least 0.3mm. The average may be less than 2mm, preferably less than 1mm, more preferably less than 0.8mm.

The ratio of the average volume of the first particles to the average volume of the second particles may be in the range 0.2 to 5, preferably in the range 0.3 to 3, more preferably in the range 0.5 to 2.

Preferably at least 90 wt%, preferably at least 95 wt%, more preferably about 100 wt% of said composition is made up of said polymeric material and bioactive material.

Said polymeric material preferably comprises a bio-compatible polymeric material. Said polymeric material preferably comprises a thermoplastic polymer.

Suitably, the polymeric material is of a type which includes:
(a) phenyl moieties;
(b) ketone moieties; and
(c) ether moieties.

Said polymeric material may have a Notched Izod Impact Strength (specimen 80mm x 10mm x 4mm with a cut 0.25mm notch (Type A), tested at 23°C, in accordance with ISO180) of at least 4KJm⁻², preferably at least 5KJm⁻², more preferably at least 6KJm⁻². Said Notched Izod Impact Strength, measured as aforesaid, may be less than 10KJm⁻², suitably less than 8KJm⁻².

The Notched Izod Impact Strength, measured as aforesaid, may be at least 3KJm⁻², suitably at least 4KJm⁻², preferably at least 5KJm⁻². Said impact strength may be less than 50 KJm⁻², suitably less than 30KJm⁻².

Said polymeric material suitably has a melt viscosity (MV) of at least 0.06 kNsm⁻², preferably has a MV of at least 0.09 kNsm⁻², more preferably at least 0.12 kNsm⁻², especially at least 0.15 kNsm⁻².

MV is suitably measured using capillary rheometry operating at 400°C at a shear rate of 1000s⁻¹ using a tungsten carbide die, 0.5x3.175mm.

Said polymeric material may have a MV of less than 1.00 kNsm⁻², preferably less than 0.5 kNsm⁻².

Said polymeric material may have a MV in the range 0.09 to 0.5 kNsm⁻², preferably in the range 0.14 to 0.5 kNsm⁻², more preferably in the range 0.3 to 0.5 kNsm⁻².

Said polymeric material may have a tensile strength, measured in accordance with ISO527 (specimen type 1b) tested at 23°C at a rate of 50mm/minute of at least 20 MPa, preferably at least 60 MPa, more preferably at least 80 MPa. The tensile strength is preferably in the range 80-110 MPa, more preferably in the range 80-100 MPa.

Said polymeric material may have a flexural strength, measured in accordance with ISO178 (80mm x 10mm x 4mm specimen, tested in three-point-bend at 23°C at a rate of 2mm/minute) of at least 50 MPa, preferably at least 100 MPa, more preferably at least 145 MPa. The flexural strength is preferably in the range 145-180MPa, more preferably in the range 145-164 MPa.

Said polymeric material may have a flexural modulus, measured in accordance with ISO178 (80mm x 10mm x 4mm specimen, tested in three-point-bend at 23°C at a rate of 2mm/minute) of at least 1 GPa, suitably at least 2 GPa, preferably at least 3 GPa, more preferably at least 3.5 GPa. The flexural modulus is preferably in the range 3.5-4.5 GPa, more preferably in the range 3.5-4.1 GPa.

Said polymeric material may be amorphous or semi-crystalline. It is preferably semi-crystalline.

The level and extent of crystallinity in a polymer is preferably measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calorimetry (DSC).

The level of crystallinity of said polymeric material may be at least 1%, suitably at least 3%, preferably at least 5% and more preferably at least 10%. In especially preferred embodiments, the crystallinity may be greater than 25%. The level of crystallinity of said polymeric material may be less than 40%.

The main peak of the melting endotherm (Tm) of said polymeric material (if crystalline) may be at least 300°C.

Disclosed is a polymeric material that may include a repeat unit of general formula 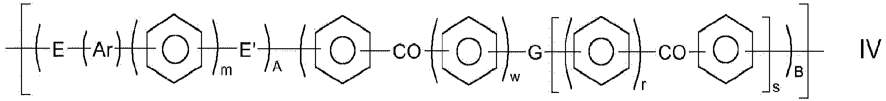 a repeat unit of general formula 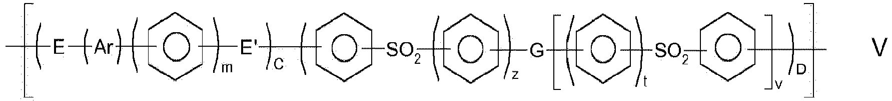 wherein A, B, C and D independently represent 0 or 1, E and E' independently represent an oxygen or a sulphur atom or a direct link, G represents an oxygen or sulphur atom, a direct link or a -O-Ph-O- moiety where Ph represents a phenyl group, m, r, s, t, v, w, and z represent zero or 1 and Ar is selected from one of the following moieties (i) to (v) which is bonded via one or more of its phenyl moieties to adjacent moieties
(i) 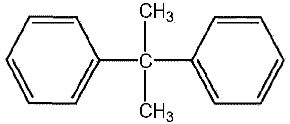
(ii) 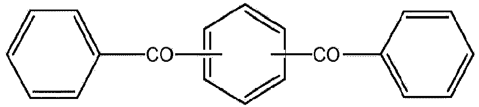
(iii) 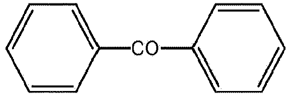
(iv) 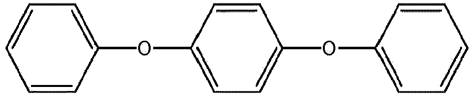
(v)

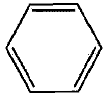

Unless otherwise stated in this specification, a phenyl moiety has 1,4-, linkages to moieties to which it is bonded.

Said polymeric material may be a homopolymer which includes a repeat unit of IV or V or may be a random or block copolymer of at least two different units of IV and/or V. Suitably in units IV and IV at least one of A or B reprsents 1; and at least one of C and D represents 1.

As an alternative to a polymeric material comprising units IV and/or V discussed above, said polymeric material may include a repeat unit of general formula 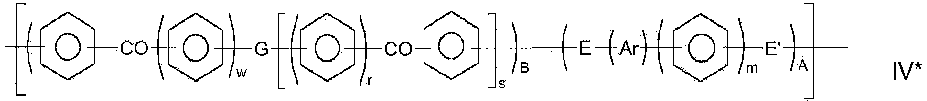
or a homopolymer having a repeat unit of general formula 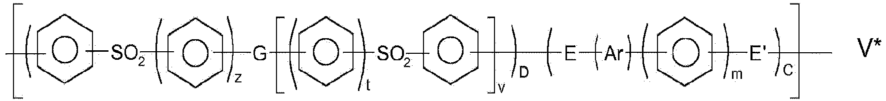
wherein A, B, C, and D independently represent 0 or 1 and E, E', G, Ar, m, r, s, t, v, w and z are as described in any statement herein.

Disclosed is a polymeric material that may be a homopolymer which includes a repeat unit of IV* or V* or a random or block copolymer of at least two different units of IV* and/or V*.

Preferably, said polymeric material is a homopolymer having a repeat unit of general formula IV.

Preferably Ar is selected from the following moieties (vi) to (x)
(vi) 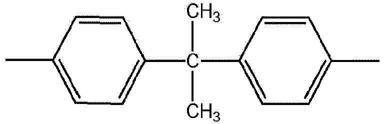
(vii) 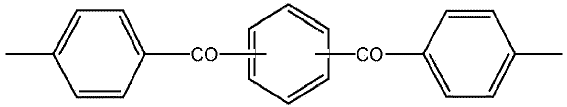
(viii) 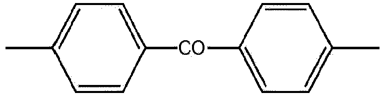
(ix) 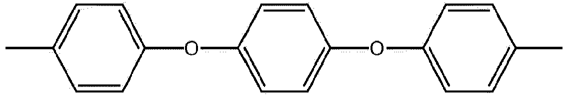
(x)

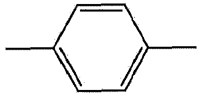

In (vii), the middle phenyl may be 1,4- or 1,3-substituted. 1,4-substituted is claimed in claim 1

Suitable moieties Ar are moieties (ii), (iii), (iv) and (v) and, of these, moieties, (ii), (iii) and (v) are disclosed. Other preferred moieties Ar are moieties (vii), (viii), (ix) and (x).
Disclosed is a class of polymeric materials that are polymers (or copolymers) which consist essentially of phenyl moieties in conjunction with ketone and/or ether moieties. That is, in the preferred class, the polymer material does not include repeat units which include -S-, -SO₂- or aromatic groups other than phenyl. Disclosed bio-compatible polymeric materials of the type described include:
(a) a polymer consisting essentially of units of formula IV wherein Ar represents moiety (v), E and E' represent oxygen atoms, m represents 0, w represents 1, G represents a direct link, s represents 0, and A and B represent 1 (i.e. polyetheretherketone).
(b) a polymer consisting essentially of units of formula IV wherein E represents an oxygen atom, E' represents a direct link, Ar represents a moiety of structure (ii), m represents 0, A represents 1, B represents 0 (i.e. polyetherketone);
(c) a polymer consisting essentially of units of formula IV wherein E represents an oxygen atom, Ar represents moiety (ii), m represents 0, E' represents a direct link, A represents 1, B represents 0, (i.e. polyetherketoneketone).
(d) a polymer consisting essentially of units of formula IV wherein Ar represents moiety (ii), E and E' represent oxygen atoms, G represents a direct link, m represents 0, w represents 1, r represents 0, s represents 1 and A and B represent 1. (i.e. polyetherketoneetherketoneketone).
(e) a polymer consisting essentially of units of formula IV, wherein Ar represents moiety (v), E and E' represents oxygen atoms, G represents a direct link, m represents 0, w represents 0, s, r, A and B represent 1 (i.e. polyetheretherketoneketone).
(f) a polymer comprising units of formula IV, wherein Ar represents moiety (v), E and E' represent oxygen atoms, m represents 1, w represents 1, A represents 1, B represents 1, r and s represent 0 and G represents a direct link (i.e. polyether-diphenyl-ether-phenyl-ketone-phenyl-).

Disclosed polymeric material may consist essentially of one of units (a) to (f) defined above. Alternatively, said polymeric material may comprise a copolymer comprising at least two units selected from (a) to (f) defined above. Disclosed copolymers include units (a). For example, a copolymer may comprise units (a) and (f); or may comprise units (a) and (e).

Disclosed polymeric material preferably comprises, more preferably consists essentially of, a repeat unit of formula (XX) 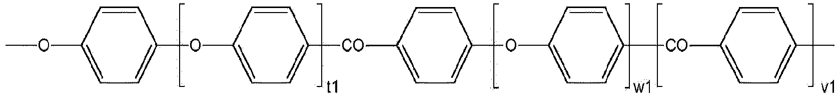 where t1, and w1 independently represent 0 or 1 and v1 represents 0, 1 or 2. Preferred polymeric materials have a said repeat unit wherein t1=1, v1=0 and w1=0; t1=0, v1=0 and w1=0; t1=0, w1=1, v1=2; or t1=0, v1=1 and w1=0. More preferred have t1=1, v1=0 and w1=0; or t1=0, v1=0 and w1=0. The most preferred has t1=1, v1=0 and w1=0 according to claim 1.

In preferred embodiments, said polymeric material is selected from polyetheretherketone, polyetherketone, polyetherketoneetherketoneketone and polyetherketoneketone. In a more preferred embodiment, said polymeric material is selected from polyetherketone and polyetheretherketone. In an especially preferred embodiment, said polymeric material is polyetheretherketone.

Said first particles may comprise said polymeric material and other optional additives, suitably so that said first particles are homogenous particles. Said first particles may comprise 40 to 100 wt% (preferably 60 to 100 wt%) of said polymeric material and 0 to 60 wt% of other additives.

Other additives may comprise reinforcing agents and may comprise additives which are arranged to improve mechanical properties of components made from the mixture. Preferred reinforcing agents comprise fibres.

80 wt%, 90 wt%, 95 wt% or about 100 wt% of said first particles are suitably made up of said polymeric material, especially a polymeric material having a repeat unit of formula (XX), especially of polyetheretherketones.

Said component may comprise a filler. The component may for example comprise a ceramic material as a filler material. The method may comprise combining polymer material, bioactive material and filler material. The method may comprise combining filler particles with particles of polymeric material and bioactive material.

Said component may include other additives, for example, reinforcing agents which may comprise additives which are arranged to improve mechanical properties of the component. Preferred reinforcing agents comprise fibres.

Said fibres may comprise a fibrous filler or a non-fibrous filler. Said fibres may include both a fibrous filler and a non-fibrous filler.

A said fibrous filler may be continuous or discontinuous. In preferred embodiments a said fibrous filler is discontinuous.

A said fibrous filler may be selected from inorganic fibrous materials, high-melting organic fibrous materials and carbon fibre.

A said fibrous filler may be selected from inorganic fibrous materials, non-melting and high-melting organic fibrous materials, such as aramid fibres, and carbon fibre.

A said fibrous filler may be selected from glass fiber, carbon fibre, asbestos fiber, silica fiber, alumina fiber, zirconia fiber, boron nitride fiber, silicon nitride fiber, boron fiber, fluorocarbon resin fibre and potassium titanate fiber. Preferred fibrous fillers are glass fibre and carbon fibre.

A fibrous filler may comprise nanofibres.

A said non-fibrous filler may be selected from mica, silica, talc, alumina, kaolin, calcium sulfate, calcium carbonate, titanium oxide, ferrite, clay, glass powder, zinc oxide, nickel carbonate, iron oxide, quartz powder, magnesium carbonate, fluorocarbon resin and barium sulfate. The list of non-fibrous fillers may further include graphite, carbon powder and nanotubes. The non-fibrous fillers may be introduced in the form of powder or flaky particles.

Preferred reinforcing agents are glass fibre and/or carbon fibre.

Other additives may comprise radiopacifiers, for example barium sulphate and any other radiopacifiers described in co-pending application PCT/GB2006/003947. Up to 20wt%, or up to 5wt% of radiopacifiers may be included. Preferably, less than 1wt%, more preferably no radiopacifier is included.

Other additives may include colourants, for example titanium dioxide. Up to 3wt% of colourant may be included but preferably less than 1wt%, more preferably no, colourant is included.

Said component may include up to 15wt%, for example up to 10wt% of other materials, that is, in addition to said polymeric material and bioactive material.

Preferably, said component consists essentially of polymeric material and bioactive material and more preferably consists essentially of a single type of polymeric material and a single type of bioactive material.

The method may include a step of altering the shape of the component. The component may be machined to alter its shape and/or to form the shape of at least part of a desired medical implant.

The method may include forming a medical implant or a part thereof.

The method may comprise producing a bioactive component which incorporates a filler material. The component may comprise a filler material.

Suitably, the filler material comprises a glass. The filler material may consist of a glass. Suitably, the filler material comprises a glass having a melting temperature higher than that of the polymeric material.

Suitably, the filler material comprises a ceramic material. The filler material may consist of a ceramic material. Suitably, the filler material comprises a ceramic material having a melting temperature higher than that of the polymeric material.

The ceramic material may be a controlled release glass. Controlled release glasses are preferably biocompatible and/or biologically inert. Said controlled release glass is preferably completely soluble in water at 38°C. On dissolution (in isolation, i.e. not when as part of said mass of material), said controlled release glass suitably has a pH of less than 7, suitably less than 6.8, preferably less than 6.5, more preferably less than 6.

Suitably, the filler material comprises a "space filler", for example salts or soluble glasses, adapted to maintain spaces between the polymeric material during the moulding stage such that the method produces a porous material. Suitably, the space filler is soluble, suitably water soluble.

The method may comprise manufacturing a component using polymeric material powder, granules, microgranules and/or particles, for example PEEK powder, granules, microgranules and/or particles.

The method may comprise manufacturing a component using polymeric material powder, granules, microgranules and/or particles, for example PEEK powder, granules, microgranules and/or particles mixed with permanent or semi-permanent fillers.

The method may include the step of treating the component to remove at least some filler material. Such treatment may be undertaken after altering the shape of the component. The treatment may be arranged to define porosity in the component.

Means for removing filler may be arranged to solubilise said filler. Said means suitably comprises a solvent. Said solvent preferably comprises water and more preferably includes at least 80wt%, preferably at least 95wt%, especially at least 99wt% water. The solvent preferably consists essentially of water.

Said ceramic material suitably has a melting point which is greater than the melting point of said polymeric material. The melting point of the ceramic material may be at least 100°C, suitably at least 200°C, preferably at least 300°C, more preferably at least 350°C greater than the melting point of said polymeric material. The melting point of the ceramic material may be at least 450°C, preferably at least 500°C, more preferably at least 600°C, especially at least 700°C.

In some embodiments, said ceramic material or part of said ceramic material may be arranged to be leached from the component in which it is incorporated, for example an implant when the implant is in situ in a human body. Said component may include a further active material which may be arranged to have a beneficial effect when liberated. For example, said active material which may be dissolved from a part, for example an implant, made from a said mass of material may comprise an active material, for example an anti-bacterial agent (e.g. silver or anti-biotic containing), a radioactive compound (e.g. which emits alpha, beta or gamma radiation for therapy, research, tracing, imaging, synovectomy or microdosimetry) or an active agent which may facilitate bone integration or other processes associated with bone (e.g. the active agent may be calcium phosphate).

The method may be used in non-medical or medical applications.

The component may comprise a part or the whole of a device which may be incorporated into or associated with a human body. Thus, the component may suitably be a part of or the whole of a medical implant. The medical implant may be arranged to replace or supplement soft or hard tissue. It may replace or supplement bone. It may be used in addressing trauma injury or craniomaxillofacial injury. It may be used in joint replacement, for example as part of a hip or finger joint replacement; or in spinal surgery.

Suitably, any desired shape may be produced. Near net-shaped ingots may be produced for further processing, for example machining; or a component which does not require any significant machining prior to use may be produced.

Also disclosed is a method of producing a component incorporating a bioactive material and which component is a medical implant and is adapted to promote bone fixation thereto, in use, and wherein the method comprises:
(a) using a screw extruder to melt a polymeric material (I) and mix the polymeric material (I) with a bioactive material (II); and
(b) making a component by moulding.

Suitably, the polymeric material (I) is of a type which includes:
(i) phenyl moieties;
(ii) ketone moieties; and
(iii) ether moieties.

Disclosed is a method of producing a component incorporating a bioactive material and which component is a medical implant and is adapted to promote bone fixation thereto, in use, and wherein the method comprises:
(a) using a screw extruder to mix a polymeric material (I) with a bioactive material (II) and melt the polymeric material (I); and
(b) making a component by moulding; and
wherein the polymeric material (I) is of a type which includes:
(i) phenyl moieties;
(ii) ketone moieties; and
(iii) ether moieties.

The bioactive material (II) comprises a phosphate.

Suitably, the polymeric material (I) is PEEK. Suitably, the bioactive material (II) is HA.

Suitably, the method comprises any feature described in relation to the first aspect. Suitably, the method comprises a method according to the first aspect.

According to a third aspect of the present invention there is provided a component manufactured according to the method of the first and/or second aspect.

Disclosed is a component comprising a composite of a polymeric material and a bioactive material, wherein the polymeric material (I) is of a type which includes:
(i) phenyl moieties;
(ii) ketone moieties; and
(iii) ether moieties; and
   wherein the composite has a tensile strength of at least 80MPa and/or a flexural strength of at least 150MPa and/or a flexural modulus of 6GPa or less and/or an impact strength of at least 5KJm⁻².

Suitably, the bioactive material (II) comprises a phosphate.

Suitably, the impact strength is determined according to ISO 180. Suitably, the impact strength is determined according to ISO 180:2000 (80mm x 10mm x 4mm, Type A notch).

Suitably, the flexural strength is determined according to ISO 178. Suitably, the flexural strength is determined according to ISO 178:2001 (2mm/minute).

Suitably, the flexural modulus is determined according to ISO 178. Suitably, the flexural modulus is determined according to ISO 178:2001 (2mm/minute).

Suitably the tensile strength is determined according to ISO 527. Suitably the tensile strength is determined according to ISO 527:1993 parts 1&2 (50mm/minute).

Suitably, the composite has a strain at break of at least 8%.

Suitably, the strain is determined according to ISO 527. Suitably, the strain is determined according to ISO 527:1993 parts 1&2 (50mm/minute).

Suitably, the component is a medical implant. Suitably, the component is a bioactive component.

The polymeric material comprises PEEK. Suitably, the polymeric material is PEEK. The bioactive material comprises HA. Suitably, the bioactive material is HA.

The component may comprise bioactive material that has been modified or doped with one or more additional chemical elements. The component may comprise bioactive material that has been modified or doped with one or more metals. The component may for example comprise HA that has been modified or doped. The HA may for example be modified or doped with one or more metals. The HA may for example be modified or doped with boron, magnesium, silicate or silver.

The component may comprise one or more of silicate (SiO₄²⁻), Borate (BO₃³⁻) and Strontium (Sr²⁺).

The component may comprise a bioactive material doped with one or more of silicate (SiO₄²⁻), Borate (BO₃³⁻) and Strontium (Sr²⁺). Suitably, the total content of silicate (SiO₄²⁻), Borate (BO₃³⁻ ) and Strontium (Sr²⁺) within the bioactive material will not exceed 10% by molarity as a cumulative value.

The component may comprise a bioactive material comprising a calcium phosphate, for example HA, in which a proportion of the calcium phosphate is substituted with one or more of silicate (SiO₄²⁻), Borate (BO₃³⁻) and Strontium (Sr²⁺). Suitably, the total substitution of calcium phosphate by silicate (SiO₄²⁻), Borate (BO₃³⁻) and Strontium (Sr²⁺) will not exceed 10% by molarity as a cumulative value. Suitably, the total substitution of calcium phosphate will not exceed 10% by molarity as a cumulative value.

The component may comprise one or more of Silicon(Si), Fluorine (F), Sulphur (S), Boron (B), Strontium (Sr), Magnesium (Mg), Silver (Ag), Barium (Ba), Zinc (Zn), Sodium (Na), Potassium (K), Aluminium (Al), Titanium (Ti)and Copper (Cu).

The component may comprise a bioactive material comprising one or more of Silicon(Si), Fluorine (F), Sulphur (S), Boron (B), Strontium (Sr), Magnesium (Mg), Silver (Ag), Barium (Ba), Zinc (Zn), Sodium (Na), Potassium (K), Aluminium (Al), Titanium (Ti) and Copper (Cu).

The component may comprise a bioactive material comprising a calcium phosphate lattice, for example a HA lattice in which single or multiple elements are introduced. For example the bioactive material may comprise a calcium phosphate lattice into which one or more of Silicon(Si), Fluorine (F), Sulphur (S), Boron (B), Strontium (Sr), Magnesium (Mg), Silver (Ag), Barium (Ba), Zinc (Zn), Sodium (Na), Potassium (K), Aluminium (Al), Titanium (Ti) and Copper (Cu) are introduced.

The bioactive material may comprise HA that has been modified or doped such that it is more potent than HA alone. This may allow lower concentrations of bioactive material to be used in the component.

The component may comprise a porous material. The component may for example comprises a material which is rendered porous using salt leaching or laser sintering.

The component may comprise a shaped object, for example a medical implant. The component may comprise a film, filament or textile.

The component may comprise a matrix in which there is substantially homogenous distribution of bioactive material, for example substantially homogenous distribution of HA or doped/modified HA. The component may comprise a matrix in which there is homogenous distribution of bioactive material, for example homogenous distribution of HA or doped/modified HA.

The component may comprise an adjunct that may help mixing and/or bonding. The adjunct may for example comprise one or more sizing agents.

The component may have a change in the surface energy and/or hydrophilicity of PEEK when compared to PEEK alone. Such a change in surface energy and/or hydrophilicity may be beneficial, for example for protein attachment.

Suitably, the polymeric material (I) comprises any feature as described in relation to the first aspect. Suitably, the bioactive material (II) comprises any feature as described in relation to the first aspect. Suitably, the composite comprises any feature as described in relation to the first aspect. Suitably, the component comprises any feature as described in relation to the first aspect.

The component may be produced according to the method of the first and/or second aspect.

Any feature of any aspect of any invention or embodiment described herein may be combined with any feature of any aspect of any invention or embodiment described herein *mutatis mutandis.*

Specific embodiments of the invention will now be described, by way of example.

### Example 1

A bioactive component was manufactured by using a screw extruder to mix a polymeric material (polyetheretherketone) with a bioactive material (hydroxyapatite) and melt the polymeric material. The extruded composite was formed into pellets which were then used to make said component by injection moulding.

Polyetheretherketone (PEEK) obtained in the form of PEEK-OPTIMA^{®} (Invibio Biomaterial Solutions, UK) was dried to remove water (it absorbs around 0.5% by weight of water during storage). The PEEK was in the form of granules of approximately 3mm by 2mm size.
The dried PEEK was mixed with hydroxyapatite (HA) obtained from Plasma Biotal Ltd., UK in the form of particles having mean particle size of 5µm.

The PEEK and HA were mixed in a twin screw compounder (extruder) which also heated the mixture to between 360°C and 400°C (with a temperature of 400°C at the extruder output) to melt the PEEK This resulted in the PEEK polymer being in the fluid state within the extruder. The PEEK was introduced to the extruder at a point upstream from the introduction of HA to the extruder. The PEEK was heated and conveyed through the extruder such that the PEEK was in a molten state within the extruder before the HA was added. The mixture of HA and molten PEEK was then conveyed further through the extruder to mix the PEEK and HA. A PEEK and HA composite was extruded from the extruder and pelletized.

The PEEK and HA were added to the extruder in a ratio such that the output of the extruder was a PEEK and HA composite which comprised 10% by weight of HA.

The extruder comprised a normal screw profile fabricated from stainless steel with a minimum L/D ratio of 45:1. At the extrusion end a twin hole die with a 4mm orifice and pelletizer was used. The main screw rotation speed was set at 150-250 rpm. The screws were intermeshing counter rotating screws having a length of around 1m and a diameter of around 40mm

The PEEK and HA composite pellets produced by the extruder were laces of approximately 2mm diameter which were chopped to lengths of approximately 3mm.. These were fed to an injection moulding machine and injection moulded to produce a bioactive component. The injection moulding machine comprised a heated barrel through which the pellets were conveyed by a screw. The barrel was heated to temperatures of between 360°C and 375°C such that the polymeric material within the pellets melted as they were conveyed through the barrel such that a melt was produced. The melt was then injected through a nozzle into a mould with the mould tool being heated to between 200°C and 220°C.

Mechanical properties, including impact strength (ISO 180), flexural strength (ISO 178), flexural modulus (ISO 178), tensile strength (ISO 527), and strain at break (ISO 527), were determined and the results are shown in Table 1.

### Example 2

The method of Example 1 was repeated but the ratio of PEEK to HA was adapted such that the output of the extruder was a PEEK and HA composite which comprised 20% by weight of HA.

The PEEK and HA composite pellets produced by the extruder were injection moulded to produce a bioactive component.

Mechanical properties, including impact strength (ISO 180), flexural strength (ISO 178), flexural modulus (ISO 178), tensile strength (ISO 527), and strain at break (ISO 527), were determined and the results are shown in Table 1.

### Example 3

The method of Example 1 was repeated but the ratio of PEEK to HA was adapted such that the output of the extruder was a PEEK and HA composite which comprised 30% by weight of HA.

The PEEK and HA composite pellets produced by the extruder were injection moulded to produce a bioactive component.

Mechanical properties, including impact strength (ISO 180), flexural strength (ISO 178), flexural modulus (ISO 178), tensile strength (ISO 527), and strain at break (ISO 527), were determined and the results are shown in Table 1.

### Example 4

The method of Example 1 was repeated but the ratio of PEEK to HA was adapted such that the output of the extruder was a PEEK and HA composite which comprised 40% by weight of HA.

The PEEK and HA composite pellets produced by the extruder were injection moulded to produce a bioactive component.

Mechanical properties, including impact strength (ISO 180), flexural strength (ISO 178), flexural modulus (ISO 178), tensile strength (ISO 527), and strain at break (ISO 527), were determined and the results are shown in Table 1.

### Example 5

The method of Example 1 was repeated but the ratio of PEEK to HA was adapted such that the output of the extruder was a PEEK and HA composite which comprised 50% by weight of HA.

The PEEK and HA composite pellets produced by the extruder were injection moulded to produce a bioactive component.

Mechanical properties, including impact strength (ISO 180), flexural strength (ISO 178), flexural modulus (ISO 178), tensile strength (ISO 527), and strain at break (ISO 527), were determined and the results are shown in Table 1.

### Comparative Example

Polyetheretherketone (PEEK) obtained in the form of PEEK-OPTIMA^{®} (Invibio Biomaterial Solutions, UK) was used in an injection moulding machine and injection moulded to produce a component corresponding structurally to that of Examples 1 to 5.

Mechanical properties, including impact strength (ISO 180), flexural strength (ISO 178), flexural modulus (ISO 178), tensile strength (ISO 527), and strain at break (ISO 527), were determined for comparison with the components of Examples 1 to 5 and the results are shown in Table 1.

PEEK was successfully compounded with HA up to 50% fill by weight, without significant issue and with no reaction observed between the two components. The mean mechanical values for impact strength, flexural strength, flexural modulus, tensile strength, and strain at break were plotted against the filler content and compared with those of the unfilled PEEK to determine optimum HA levels.

From this it was concluded that 20% by weight of HA (Example 2) gave the optimum level to allow HA to be present at sufficient levels to provide desirable bioactivity to the component without significant detriment to the physical properties.

| TABLE 1 | | | | | | |
|---|---|---|---|---|---|---|
| Property | Comparative Example (No HA) | Example 1 (10% HA) | Example 2 (20% HA) | Example 3 (30% HA) | Example 4 (40% HA) | Example 5 (50% HA) |
| Impact Strength (KJ/m2) | 7.33 | 7.4 | 6.1 | 5.2 | 4.6 | 4.6 |
| Flexural strength (MPa) | 162.45 | 156.1 | 156.0 | 154.2 | 139.2 | 118.8 |
| Flexural modulus (GPa) | 3.96 | 4.33 | 4.72 | 5.61 | 6.67 | 8.02 |
| Tensile Strength (MPa) | 99.25 | 88.7 | 88.7 | 81.8 | 73.5 | 75.5 |
| Strain at Break (%) | 35.8 | 24.09 | 8.8 | 3.98 | 2.24 | 1.27 |

### Bioactivity tests

PEEK containing 20% by weight HA (Example 2) was chosen for further bioactivity studies due to the limited effects on material mechanical properties compared to PEEK alone (comparative example).

Bioactivity of the PEEK/HA was determined by the ability to form apatite on the surface of the material in a simulated body fluid SBF using SBF-JL2 as prepared and described in Bohner and Lemaitre (Bohner M, Lemaitre J. / Biomaterials 30 (2009) 2175-2179) and compared with PEEK controls.

The SBF-JL2 was produced using a dual-solution preparation (Sol. A and Sol.B) having the following composition for 2L of final fluid:

| Starting Materials | | | Weights of starting materials [g/L] | |
|---|---|---|---|---|
| Formula | MW [g/mol] | Purity [-] | Sol. A | Sol. B |
| NaCl | 58.44 | 99.5% | 6.129 | 6.129 |
| NaHCO₃ | 84.01 | 99.5% | 5.890 | |
| Na₂HPO₄.2H₂O | 177.99 | 99.0% | 0.498 | |
| CaCl₂ | 110.99 | 95.0% | | 0.540 |
| | | | Volume of HCl solution (mL/L) | |
| HCl 1.00 M | Aq. Sol. | [mL/L] | 0.934 | 0.934 |

Use of this in vitro method of examining apatite formation as a means of predicting in vivo bone bioactivity is both widely used and accepted (Kokubo T, Takadama H. How useful is SBF in predicting in vivo bone bioactivity? Biomaterials 2006;27(15):2907-2915) and Bohner and Lemaitre relates to a variant method. Samples were immersed in SBF for 1, 3 and 7 days on a rotating platform at 37°C with 5% CO₂ and 100% humidity. X-ray photoelectron spectroscopy (XPS), scanning electron microscopy (SEM), and attenuated total reflectance Fourier transform infrared spectroscopy (ATR-FTIR) were used to analyze the bioactive elements present on the surface of the specimens following immersion in SBF.

SEM analysis of the surface of PEEK controls and PEEK/20%HA composite revealed the formation of spherical crystals on the surface after immersion in SBF. These were more numerous and apparent on the PEEK/20%HA samples and these were observed as early as 1 day post-immersion in SBF, suggesting increased apatite formation.

Detailed Ca2p and P2p XPS spectra revealed that although Ca and P were identified on the surface of both materials, only elemental ratios present on the PEEK/20%HA samples were conducive to bone formation with a Ca/P ratio of 1.66, close to the theoretical value for hydroxyapatite. Meanwhile, the ratios of the depositions on the control PEEK were more variable (>1.67), and indicative of non-hydroxyapatite calcium phosphate formations.

Following immersion in SBF for 1 day, ATR-FTIR surface analysis was performed on PEEK/20%HA and control PEEK samples to semi-quantify the degree of apatite deposition and detect functional groups. A significant peak was observed at 1015 cm⁻¹, most likely arising from the structural P-O bond of phosphate groups. The ratio of absorption at 1015cm⁻¹ to 1645cm⁻¹ (characteristic of PEEK) was measured and showed an increased ratio on PEEK/20%HA samples compared with control PEEK, confirming the XPS findings indicating greater apatite formation on the PEEK/20%HA samples.

Surprisingly it has been found that despite the low proportion of HA in the component (only 20% by weight) sufficient HA is available at the surface of the component to impart bioactive properties to the component and promote apatite formation. Without wishing to be bound by theory it is believed that the surface availability of HA and the effectiveness of the low level of HA in promoting apatite formation is due to the use of a screw extrusion method to produce PEEK and HA composite pellets. This is an unexpected effect of using a screw extrusion and pelletization process.

It will be appreciated that preferred embodiments of the present invention may allow the manufacture of a bioactive component which comprises a polymeric material incorporating a bioactive material and which may benefit from bioactive properties of the bioactive material whilst retaining desirable physical properties of the polymeric material.

The compounding of PEEK with HA according to preferred embodiments of the present invention may produce bioactive components that are unexpected in a number of ways:
The components produced may still be mechanically strong.

Examples with lower levels of HA (such as example 2 with 20% by weight of HA) may retain most of the properties of PEEK, yet the dispersion at the surface may show uniformity and a lot of HA presence.

The components may be bioactive. For example when the component comprising 20% by weight HA was placed in a simulated body fluid test for bioactivity, it passed that test with the formation of new apatite (CaP) at the ratio of 1.66 (theoretical value for HA ratio is 1.67) versus controls that did not have this ratio.

The HA:PEEK interface may be good. This has been a short-coming of previous processes.

The HA may show up near the surface at good uniformity and in sufficient amounts to confer bioactivity.

A relatively low concentration of HA (for example 20% by weight) may create a sweet spot of mechanical strength and bioactivity whilst retaining the flexibility of using industrial relevant manufacturing methods.

The bioactivity conferred may be afforded by a compound that may be created without any adverse reactions.

## Claims

1. A method of producing a component incorporating a bioactive material wherein the method comprises:
(a) using a twin-screw extruder to mix a polymeric material (I) with a bioactive material (II) and melt the polymeric material (I); and
(b) making a component by moulding; and
wherein the polymeric material (I) comprises polyetheretherketone (PEEK);
wherein the bioactive material (II) comprises hydroxyapatite
wherein the component comprises polymeric material (I) in an amount of between 75% and 85% by weight of the component and bioactive material (II) in an amount of between 15% and 25% by weight of the component; and
wherein the component comprises a polymeric material-bioactive material composite having a tensile strength of at least 85 MPa.

2. A method according to any preceding claim, wherein the component consists of polyetheretherketone and hydroxyapatite.

3. A method according to any preceding claim, wherein at the extrusion end of the extruder, the extruder has a pelletizer.

4. A method according to any preceding claim, wherein the method comprises producing pellets having a diameter of 3.0mm or less.

5. A method according to any preceding claim, wherein the method comprises producing pellets of composite material in step (a) and making a part by moulding from the pellets in step (b).

6. A method according to any preceding claim, wherein step (b) comprises injection moulding.

7. A method according to any preceding claim, wherein: the component is a component which, when placed in a simulated body fluid (SBF) test for bioactivity, passes said test with the formation of new apatite (CaP) at the ratio close to the theoretical value for hydroxyapatite, which is 1.67.

8. A method according to any preceding claim, wherein the method comprises producing a component comprising a polymeric material-bioactive material composite having tensile strength and/or flexural strength which are at least 80% of the respective strength of the polymeric material; and or
the method comprises producing a component comprising a polymeric material-bioactive material composite having a tensile strength which is at least 85% of the respective strength of the polymeric material.

9. A method according to any preceding claim, wherein the method comprises introducing the bioactive material to the extruder at a point downstream of the point at which the polymeric material is introduced to the extruder; and preferably the method comprises introducing the bioactive material to the extruder at a point at which the polymeric material is at least partially molten.

10. A method according to any preceding claim, wherein the extruder is a twin screw extruder having screws of between 20mm and 50mm in diameter.

11. A method according to any preceding claim, wherein the extruder has counter rotating intermeshing screws.

12. A method according to any preceding claim, wherein the method uses a main screw rotation speed of between 150 and 250rpm; and/or
wherein the size and output of the extruder are matched to obtain a residence time of from 3 to 12 minutes.

13. A method as claimed in any one of the preceding claims wherein the component comprises a porous material comprising a material which is rendered porous using salt leaching or laser sintering.

14. Pellets comprising 75 to 85% by weight of polyetheretherketone and 15 to 25 wt% of hydroxyapatite, wherein said pellets define a composite material having the following properties:
a tensile strength of at least 80 MPa, when measured in accordance with ISO 527;
a flexural strength of at least 150 MPa, when measured in accordance with ISO 178;
a flexural modulus of 6 GPa or less, when measured in accordance with ISO 178;
an impact strength of at least 5KJ m⁻², when measured in accordance with ISO 180;
a strain at break of at least 8%, when measured in accordance with ISO 527.

## Patentansprüche

1. Verfahren zum Herstellen einer Komponente, die ein bioaktives Material aufnimmt, wobei das Verfahren Folgendes umfasst:
(a) Verwenden eines Doppelschneckenextruders zum Mischen eines Polymermaterials (I) mit einem bioaktiven Material (II) und Schmelzen des Polymermaterials (I); und
(b) Fertigen einer Komponente durch ein Formen; und
wobei das Polymermaterial (I) Polyetheretherketon (PEEK) umfasst,
wobei das bioaktive Material (II) Hydroxylapatit umfasst, wobei die Komponente Polymermaterial (I) in einer Menge zwischen 75 Gew.-% und 85 Gew.-% der Komponente und bioaktives Material (II) in einer Menge zwischen 15 Gew.-% und 25 Gew.-% der Komponente umfasst, und
wobei die Komponente einen Verbundstoff aus Polymermaterial und bioaktivem Material, der eine Zugfestigkeit von wenigstens 85 MPa aufweist, umfasst.

2. Verfahren nach einem vorhergehenden Anspruch, wobei die Komponente aus Polyetheretherketon und Hydroxylapatit besteht.

3. Verfahren nach einem vorhergehenden Anspruch, wobei der Extruder an dem Extrusionsende des Extruders einen Pelletizer aufweist.

4. Verfahren nach einem vorhergehenden Anspruch, wobei das Verfahren ein Herstellen von Granulat, das einen Durchmesser von höchstens 3,0 mm aufweist, umfasst.

5. Verfahren nach einem vorhergehenden Anspruch, wobei das Verfahren das Herstellen von Granulat aus Verbundstoffmaterial in Schritt (a) und das Fertigen eines Teils durch das Formen aus dem Granulat in Schritt (b) umfasst.

6. Verfahren nach einem vorhergehenden Anspruch, wobei Schritt (b) ein Spritzgießen umfasst.

7. Verfahren nach einem vorhergehenden Anspruch, wobei:
die Komponente eine Komponente ist, die, wenn sie in einen Test mit simulierter Körperflüssigkeit (*simulated body fluid* - SBF) auf Bioaktivität platziert wird, den Test mit der Ausbildung von neuem Apatit (CaP) in einem Verhältnis nahe dem theoretischen Wert für Hydroxylapatit, der 1,67 beträgt, besteht.

8. Verfahren nach einem vorhergehenden Anspruch, wobei das Verfahren das Herstellen einer Komponente umfasst, umfassend einen Verbundstoff aus Polymermaterial und bioaktivem Material, der eine Zugfestigkeit und/oder Biegefestigkeit wenigstens 80 % der jeweiligen Festigkeit des Polymermaterials beträgt, aufweist, und oder
das Verfahren das Herstellen einer Komponente umfasst, umfassend einen Verbundstoff aus Polymermaterial und bioaktivem Material, der eine Zugfestigkeit aufweist, die wenigstens 85 % der jeweiligen Festigkeit des Polymermaterials beträgt.

9. Verfahren nach einem vorhergehenden Anspruch, wobei das Verfahren ein Einbringen des bioaktiven Materials in den Extruder an einem Punkt stromabwärts des Punktes umfasst, an dem das Polymermaterial in den Extruder eingebracht wird, und vorzugsweise das Verfahren das Einbringen des bioaktiven Materials in den Extruder an einem Punkt umfasst, an dem das Polymermaterial wenigstens teilweise geschmolzen ist.

10. Verfahren nach einem vorhergehenden Anspruch, wobei der Extruder ein Doppelschneckenextruder ist, der Schnecken mit einem Durchmesser zwischen 20 mm und 50 mm aufweist.

11. Verfahren nach einem vorhergehenden Anspruch, wobei der Extruder gegenläufige ineinandergreifende Schnecken aufweist.

12. Verfahren nach einem vorhergehenden Anspruch, wobei das Verfahren eine Hauptschneckendrehzahl zwischen 150 und 250 U/min verwendet; und/oder wobei die Größe und die Leistung des Extruders aufeinander abgestimmt sind, um eine Verweilzeit von 3 bis 12 Minuten zu erhalten.

13. Verfahren nach einem vorhergehenden Anspruch, wobei die Komponente ein poröses Material umfasst, umfassend ein Material, das unter Verwendung von Salzlaugung oder Lasersintern porös gemacht wird.

14. Granulat, umfassend 75 bis 85 Gew.-% Polyetheretherketon und 15 bis 25 Gew.-% Hydroxylapatit, wobei das Granulat ein Verbundstoffmaterial definiert, das die folgenden Eigenschaften aufweist:
eine Zugfestigkeit von wenigstens 80 MPa, wenn gemäß ISO 527 gemessen;
eine Biegefestigkeit von wenigstens 150 MPa, wenn gemäß ISO 178 gemessen;
ein Biegemodul von höchstens 6 GPa, wenn gemäß ISO 178 gemessen;
eine Schlagfestigkeit von wenigstens 5KJm⁻², wenn gemäß ISO 180 gemessen;
eine Bruchdehnung von wenigstens 8 %, wenn gemäß ISO 527 gemessen.

## Revendications

1. Procédé de production d'un constituant incorporant un matériau bioactif, le procédé comprenant :
(a) l'utilisation d'une extrudeuse à deux vis pour mélanger un matériau polymère (I) avec un matériau bioactif (II) et pour faire fondre le matériau polymère (I) ; et
(b) la fabrication d'un constituant par moulage ; et
dans lequel le matériau polymère (I) comprend de la polyétheréthercétone (PEEK) ; dans lequel le matériau bioactif (II) comprend de l'hydroxyapatite dans lequel le constituant comprend un matériau polymère (I) en une quantité comprise entre 75 % et 85 % en poids du constituant et un matériau bioactif (II) en une quantité comprise entre 15 % et 25 % en poids du constituant ; et
dans lequel le constituant comprend un composite de matériau polymère-matériau bioactif ayant une résistance à la traction d'au moins 85 MPa.

2. Procédé selon l'une quelconque des revendications précédentes, dans lequel le constituant est constitué de polyétheréthercétone et d'hydroxyapatite.

3. Procédé selon l'une quelconque des revendications précédentes, dans lequel, à l'extrémité d'extrusion de l'extrudeuse, l'extrudeuse comporte un granulateur.

4. Procédé selon l'une quelconque des revendications précédentes, le procédé comprenant la production de pastilles ayant un diamètre de 3,0 mm ou moins.

5. Procédé selon l'une quelconque des revendications précédentes, le procédé comprenant la production de pastilles de matériau composite à l'étape (a) et la fabrication d'une pièce par moulage à partir des pastilles à l'étape (b).

6. Procédé selon l'une quelconque des revendications précédentes, dans lequel l'étape (b) comprend le moulage par injection.

7. Procédé selon l'une quelconque des revendications précédentes, dans lequel :
le constituant est un constituant qui, lorsqu'il est placé dans un test de bioactivité de liquide organique simulé (SBF), réussit ledit test avec la formation de nouvelle apatite (CaP) dans un rapport proche de la valeur théorique pour l'hydroxyapatite, qui est de 1,67.

8. Procédé selon l'une quelconque des revendications précédentes, le procédé comprenant la production d'un constituant comprenant un composite de matériau polymère-matériau bioactif présentant une résistance à la traction et/ou à la flexion qui représentent au moins 80 % de la résistance respective du matériau polymère ; et/ou
le procédé comprend la production d'un constituant comprenant un composite de matériau polymère-matériau bioactif ayant une résistance à la traction qui représente au moins 85 % de la résistance respective du matériau polymère.

9. Procédé selon l'une quelconque des revendications précédentes, le procédé comprenant l'introduction du matériau bioactif dans l'extrudeuse à un point en aval du point auquel le matériau polymère est introduit dans l'extrudeuse ; et
de préférence, le procédé comprend l'introduction du matériau bioactif dans l'extrudeuse à un point auquel le matériau polymère est au moins partiellement fondu.

10. Procédé selon l'une quelconque des revendications précédentes, dans lequel l'extrudeuse est une extrudeuse à deux vis ayant des vis de diamètre compris entre 20 et 50 mm.

11. Procédé selon l'une quelconque des revendications précédentes, dans lequel l'extrudeuse comporte des vis d'engrènement contrarotatives.

12. Procédé selon l'une quelconque des revendications précédentes, dans lequel le procédé utilise une vitesse de rotation de la vis principale comprise entre 150 et 250 tr/min ; et/ou dans lequel la taille et le débit de l'extrudeuse sont adaptés pour obtenir un temps de séjour allant de 3 à 12 minutes.

13. Procédé selon l'une quelconque des revendications précédentes, dans lequel le constituant comprend un matériau poreux comprenant un matériau qui est rendu poreux au moyen d'une lixiviation au sel ou d'un frittage laser.

14. Pastilles comprenant de 75 à 85 % en poids de polyétheréthercétone et de 15 à 25 % en poids d'hydroxyapatite, lesdites pastilles définissant un matériau composite ayant les propriétés suivantes :
une résistance à la traction d'au moins 80 MPa, lorsqu'elle est mesurée conformément à la norme ISO 527 ;
une résistance à la flexion d'au moins 150 MPa, lorsqu'elle est mesurée conformément à la norme ISO 178 ;
un module d'élasticité en flexion inférieur ou égal à 6 GPa, lorsqu'il est mesuré conformément à la norme ISO 178 ;
une résistance aux chocs d'au moins 5KJm⁻², lorsqu'elle est mesurée conformément à la norme ISO 180 ;
une contrainte à la rupture d'au moins 8 %, lorsqu'elle est mesurée conformément à la norme ISO 527.
